# EUROPÄISCHE PATENTSCHRIFT

(11) **EP 3 073 581 B1**
(45) Veröffentlichungstag und Bekanntmachung des Hinweises auf die Patenterteilung: **11.07.2018**
(21) Anmeldenummer: 15161479.9
(22) Anmeldetag: 27.03.2015
(51) Int. Cl.: H01R 13/631, H01R 13/24, A61L 9/20, H01R 13/74, F24C 15/20

(54) **Abluftbehandlungssystem**
Exhaust air treatment system
Système de traitement d'air pollué

(43) Veröffentlichungstag der Anmeldung: 28.09.2016
(73) Patentinhaber: Franke Technology and Trademark Ltd, 6052 Hergiswil (CH)
(72) Erfinder: Krenz, Karlheinz, 99102 Klettbach (DE)
(74) Vertreter: Lemcke, Brommer & Partner Patentanwälte Partnerschaft mbB

(56) Entgegenhaltungen:
- EP-A2- 2 345 916
- WO-A2-2004/011316
- DE-A1- 19 711 376
- DE-U1-202012 100 351
- US-A- 4 519 667
- US-A- 5 879 185
- US-A1- 2010 200 280
- US-B1- 8 357 000

## Beschreibung

Die Erfindung betrifft ein Abluftbehandlungssystem, insbesondere für Großküchen, gemäß dem Oberbegriff des Anspruchs 1 mit einer steckerlosen elektrischen Kontakteinrichtung.

Aus der WO2012/051987 A1 ist eine Vorrichtung zur Abluftreinigung mittels UV-Strahlung bekannt, bei der eine spezielle wasserdichte Steckverbindung vorgesehen ist, um ein Trägergestell der Vorrichtung, welches die UV-Lampen umfasst, in einer Spülmaschine reinigen zu können. Die Steckverbindung selbst ist in dem genannten Dokument allerdings nur recht oberflächlich beschrieben.

Weitere UV-basierte Vorrichtungen, insbesondere zur Abluftreinigung, sind aus der US 5,660,719 A, der WO 2009/092546 A2, der DE 10 2005 043 605 A1, der DE 601 33 086 T2 und der WO 94/08633 A1 bekannt. Insbesondere aus der DE 601 33 086 T2 ist zum Anschluss einer UV-Kassette mit UV-Röhren eine herkömmliche, lösbare Steckverbindung mit einem Kabel vorgesehen, das vor Entnahme der Kassette zu entfernen und vor erneuter Inbetriebnahme wieder anzubringen ist.

US 8,357,000 B1 offenbart eine fluiddichte elektrische Kontakteinrichtung für den Einsatz bei Kanonen oder dgl. mit zwei Kontaktträgern, von denen der eine an einer durch Rückschlag beweglichen Trägereinrichtung gelagert ist, während der andere starr an einer anderen Trägereinrichtung gelagert ist.

US 2010/200280 A1 offenbart eine Anschlussvorrichtung für eine Leiterplatte, bei der Federelemente vorgesehen sind, um eine mechanische und/oder elektrische Verbindung herzustellen.

Aus der DE 197 11 376 A1 ist eine elektrische Steckverbindung bekannt, die gegenüber ungünstigen Umgebungsbedingungen eine sichere Kontaktgabe gewährleisten soll. Dazu sind insbesondere Zwischenkontakte aus einem Drahtgeflecht vorgesehen, die zwischen Kontaktstiften einerseits und Steckerkontakten andererseits angeordnet sind.

Aus der EP 2 345 916 A2 sind elektrische Verbindungsanordnungen mit mehreren Kontaktträgern bekannt, die zwischen eingefahrenen und ausgefahrenen Stellungen bewegt werden, um in der ausgefahrenen Stellung mit einer ausgewählten Komponente gekoppelt zu werden.

Die WO 2004/011316 offenbart eine drahtlose elektrische Verbindung für Komponenten, die an einem beweglichen Tragesystem eines Lastkraftfahrzeugs angeordnet sind.

Die DE 20 2012 100 351 U1 zeigt UV-Module für eine gewerblich nutzbare Küchenluftreinigungsanlage. Dort ist ein UV-Lichtmodul beschrieben, das über eine nicht näher erläuterte Stecker-/Gegenstecker-Verbindung elektrisch an ein UV-Reinigungsmodul der Küchenluftreinigungsanlage angeschlossen wird.

Es hat sich in der Vergangenheit wiederholt gezeigt, dass ein Vorsehen derartiger Anschlusseinrichtungen in Form von Steckverbindungen in der betrieblichen Hektik dazu führt, dass die UV-Einrichtungen nicht wieder korrekt in Betrieb genommen oder gleich gar nicht entnommen und entsprechend auch nicht gereinigt werden. Dies führt zu einer übermäßigen Verschmutzung des gesamten Abluftbehandlungssystems verbunden mit einem erhöhten Wartungs- und Reinigungsaufwand sowie einer verstärkten Umweltbelastung. Außerdem ist gerade in Großküchen oftmals schlecht eingelerntes Personal mit der Reinigung derartiger Abluftbehandlungssysteme betraut, sodass die Gefahr von Beschädigungen aufgrund unsachgemäßer oder fehlerhafter Handhabung besteht.

Es besteht also Bedarf an einem Abluftbehandlungssystem mit einer elektrischen Kontakteinrichtung, bei dem der Aufwand für die Handhabung miniert und die Gefahr von fehlerhaftem Gebrauch praktisch ausgeschlossen ist. Zudem soll sich das Abluftbehandlungssystem für eine einfache Reinigung in einer Spülmaschine eignen und eine sichere elektrische Kontaktierung im Betrieb gewährleisten.

Die Aufgabe wird gelöst durch ein Abluftbehandlungssystem mit den Merkmalen des Anspruchs 1. Vorteilhafte Weiterbildungen sind Gegenstand von Unteransprüchen.

Bei einem erfindungsgemäßen Abluftbehandlungssystem, insbesondere für Großküchen, mit wenigstens einem Trageteil und mit wenigstens einem an dem Trageteil angeordneten oder anordbaren, abnehmbaren Gehäuseteil, welches Gehäuseteil im Betrieb des Abluftbehandlungssystems von Abluft durchströmt oder durchströmbar ist und in welchem Gehäuseteil wenigstens eine UV-Einheit angeordnet ist, durch die die Abluft mit elektromagnetischer Strahlung im UV-Bereich beaufschlagbar ist, wobei an dem Trageteil und an dem Gehäuseteil gemeinsam wenigstens eine Kontakteinrichtung angeordnet ist, die folgendes umfasst: einen ersten Kontaktträger mit einer ersten Anzahl erster Kontaktelemente; einen zweiten Kontaktträger mit einer zweiten Anzahl zweiter Kontaktelemente; und eine Federkrafteinrichtung, die zum Einwirken auf den zweiten Kontaktträger ausgebildet ist; wobei wenigstens eine Unteranzahl der ersten Kontaktelemente oder der zweiten Kontaktelemente derart angeordnet ist, dass sie gemeinsam mit jeweils einem Kontaktelement an dem anderen Kontaktträger paarweise in elektrisch leitenden Kontakt gebracht oder bringbar sind, um in einem verbundenen Zustand der Kontakteinrichtung Kontaktelementpaare zu bilden; wobei die ersten Kontaktelemente oder die zweiten Kontaktelemente als Federkontakte ausgebildet sind, deren Spitzen zumindest in einem unverbundenen Zustand der Kontakteinrichtung um ein Übermaß über eine Oberfläche des betreffenden Kontaktträgers hinausragen, welche Oberfläche zumindest in dem verbundenen Zustand dem anderen Kontaktträger zugewandt ist; wobei durch die Federkrafteinrichtung eine Federkraft auf den zweiten Kontaktträger in Richtung auf den ersten Kontaktträger bewirkt oder bewirkbar ist, wenn die genannten Kontaktelementpaare gebildet sind; wobei der erste Kontaktträger im Wesentlichen unverschiebbar an einer ersten Trageinrichtung befestigt ist, welche erste Trageinrichtung an dem Trageteil angeordnet ist oder durch dieses Trageteil gebildet ist; wobei der zweite Kontaktträger an einer zweiten Trageinrichtung befestigt und gegenüber dieser zweiten Trageinrichtung gegen die durch die Federkrafteinrichtung bewirkte oder bewirkbare Federkraft verschiebbar ist, welche zweite Trageinrichtung an dem Gehäuseteil angeordnet ist oder durch dieses Gehäuseteil gebildet ist; und wobei der verbundene Zustand der Kontakteinrichtung erreicht wird beim Anordnen des Gehäuseteil an dem Trageteil.

Die elektrische Kontakteinrichtung ist als steckerlose Kontakteinrichtung ausgeführt und sorgt aufgrund der Ausbildung der ersten oder der zweiten Kontaktelemente als Federkontakte sowie aufgrund des Vorsehens der Federkrafteinrichtung dafür, dass aufgrund der wirksamen Federkraft eine quasi selbsttätige, sichere Bildung der Kontaktelementpaare erfolgt, wobei Fehlbedienungen praktisch ausgeschlossen sind. Außerdem ist die so geschaffene Kontakteinrichtung und entsprechend auch das Abluftbehandlungssystem optimal für eine einfache Reinigung, insbesondere des Gehäuseteils und insbesondere in einer Spülmaschine, geeignet.

Bei einer Weiterbildung des erfindungsgemäßen Abluftbehandlungssystems kann vorgesehen sein, dass der erste Kontaktträger und der zweite Kontaktträger in dem verbundenen Zustand jeweils mit wenigstens einer Teilfläche ihrer jeweiligen Oberflächen aneinander anliegen. In diesem Kontext kann weiter vorgesehen sein, dass der erste Kontaktträger und/oder der zweite Kontaktträger im Bereich der jeweiligen Teilfläche eine vorspringende Struktur und/oder ein Dichtelement aufweist. Die vorspringende Struktur und/oder das Dichtelement kann um die jeweiligen ersten und/oder zweiten Kontaktelemente umlaufend ausgebildet sein, vorzugsweise geschlossen. Dadurch lässt sich der Kontaktbereich, in dem die Kontaktelementpaare gebildet werden, gegenüber äußeren Einflüssen, insbesondere auch Feuchtigkeit, abdichten.

Es kann jedoch auch vorgesehen sein, den Kontaktbereich explizit von außen zugänglich auszubilden, um ggf. dort vorhandene Restfeuchte abführen zu können.

Wenn die vorspringende Struktur und/oder das Dichtelement an demjenigen Kontaktträger angeordnet ist, der nicht zur Reinigung insbesondere in einer Spülmaschine vorgesehen ist, oder wenn eine vorspringende Struktur und/oder das Dichtelement ganz fehlt, kann sich der Vorteil ergeben, dass im Bereich der Kontaktelemente nach erfolgter Reinigung keine Restfeuchte stehen bleibt.

Vorzugsweise ist weiterhin vorgesehen, dass die vorspringende Struktur und/oder das Dichtelement in dem verbundenen Zustand eine Höhe gegenüber einer restlichen Oberfläche des jeweiligen Kontaktträgers aufweist, welche Höhe kleiner ist als das Übermaß der Federkontakte zuzüglich eines etwaigen Übermaßes der Kontaktelemente des anderen Kontaktträgers gegenüber dessen Oberfläche, welche Oberfläche zumindest in dem verbundenen Zustand der Oberfläche des die Federkontakte aufweisenden Kontaktträgers zugewandt ist. Dadurch kommt es in dem verbundenen Zustand in jedem Fall zu einer sicheren Kontaktierung zwischen den betreffenden Kontaktelementen, ohne dass die vorspringende Struktur und/oder das Dichtelement hierbei störend wirken könnte.

Bei einer anderen Weiterbildung des erfindungsgemäßen Abluftbehandlungssystems kann vorgesehen sein, dass die nicht als Federkontakte ausgebildeten Kontaktelemente, die vorzugsweise an dem zweiten Kontaktträger angeordnet sind, im Wesentlichen flächenbündig mit der Oberfläche des betreffenden Kontaktträgers in diesem eingebettet sind. Diese Ausgestaltung ist insbesondere dann von Vorteil, wenn die nicht als Federkontakte ausgebildeten Kontaktelemente sich an dem zweiten Kontaktträger befinden und wenn dieser zudem an einem Teil des Abluftbehandlungssystems angeordnet oder anzuordnen ist, welcher Teil insbesondere in einer Spülmaschine gereinigt werden soll, beispielsweise dem erwähnten Gehäuseteil.

Der erste Kontaktträger ist erfindungsgemäß im Wesentlichen fest und unverschiebbar an der ersten Trageinrichtung befestigt, welche erste Trageinrichtung ihrerseits bereits Teil eines Abluftbehandlungssystems sein kann, beispielsweise in Form eines Trageteils oder Tragerahmens. Dagegen ist der zweite Kontaktträger erfindungsgemäß an der zweiten Trageinrichtung befestigt und gegenüber dieser Trageinrichtung entgegen der durch die Federkrafteinrichtung bewirkten oder bewirkbaren Federkraft verschiebbar. Auch die genannte zweite Trageinrichtung kann ihrerseits Teil eines Abluftbehandlungssystems sein, beispielsweise in Form eines abnehmbaren Gehäuseteils oder dgl., im welchem Gehäuseteil die angesprochenen UV-Einheiten zur Abluftbehandlung angeordnet sein können.

Um eine mechanische Stabilität der Anordnung zu gewährleisten, kann vorgesehen sein, dass zumindest in dem verbundenen Zustand die erste Trageinrichtung und die zweite Trageinrichtung bereichsweise flächig aneinander anliegen. Um außerdem die elektrische Kontaktierung zu erleichtern, kann weiterhin vorgesehen sein, dass der erste Kontaktträger oder der zweite Kontaktträger gegenüber einer Fläche der betreffenden Trageinrichtung vorspringend angeordnet ist, während der andere Kontaktträger gegenüber einer Fläche der betreffenden Trageinrichtung zurückversetzt angeordnet ist. Dies dient auch zum Schutz insbesondere des zurückversetzt angeordneten Kontaktträgers vor mechanischer Schädigung.

In diesem Kontext kann weiterhin vorgesehen sein, dass der erste Kontaktträger und der zweite Kontaktträger im Bereich von Durchbrüchen in der jeweiligen Trageinrichtung angeordnet sind.

Um auch den anderen Kontaktträger vor mechanischer Schädigung zu schützen, kann weiterhin vorgesehen sein, dass an derjenigen Trageinrichtung, an welcher Trageinrichtung der betreffende Kontaktträger gegenüber der Fläche der betreffenden Trageinrichtung vorspringend angeordnet ist, in Randbereichen des jeweiligen Durchbruchs Abkantungen vorgesehen sind, welche Abkantungen in eine gleiche Richtung und vorzugsweise zumindest um ein gleiches Maß vorspringen wie der betreffende Kontaktträger.

Weiterhin kann vorgesehen sein, dass ein Übermaß der Abkantungen gegenüber der Fläche der betreffenden Trageinrichtung gleich groß oder größer ist wie bzw. als ein Maß des Vorspringens des betreffenden Kontaktträgers gegenüber der Fläche der betreffenden Trageinrichtung, und dass ein Übermaß der Abkantungen gegenüber der Fläche der betreffenden Trageinrichtung gleich groß oder kleiner ist wie bzw. als ein Maß des Rückversatzes des anderen Kontaktträgers gegenüber der Fläche der anderen Trageinrichtung. Auf diese Weise lassen sich die Kontaktträger und die Kontaktelemente optimal schützen.

Eine wieder andere Weiterbildung der Kontakteinrichtung sieht vor, dass zumindest in dem verbundenen Zustand die erste Trageinrichtung und die zweite Trageinrichtung lösbar aneinander festgelegt sind, um so über ein Lösen der Trageinrichtungen voneinander auch die Kontakteinrichtung zu trennen.

Dabei kann weiter vorgesehen sein, dass an der ersten Trageinrichtung und/oder an der zweiten Trageinrichtung wenigstens eine Ausricht- und Fixiereinrichtung vorgesehen ist, vorzugsweise umfassend eine Rast- oder Schnappvorrichtung, beispielsweise mit Feder-Vorspannung, welche dazu ausgebildet ist, die Kontakteinrichtung in dem verbundenen Zustand zu halten, höchst vorzugsweise im Wesentlichen selbsttätig beim Annähern der ersten Trageinrichtung und der zweiten Trageinrichtung. Beispielsweise kann die Ausricht- und Fixiereinrichtung ein Element (z.B. eine Sperrklinke) aufweisen, welches beim Annähern der ersten Trageinrichtung und der zweiten Trageinrichtung gegen die Feder-Vorspannung verschoben wird, um dann nach erfolgtem Anliegen der ersten Trageinrichtung an der zweiten Trageinrichtung einzurasten und für eine Sicherung des verbundenen Zustands zu sorgen. Eine Bedienperson kann die Ausricht- und Fixiereinrichtung leicht wieder lösen, um die Kontakteinrichtung zu trennen.

Die erste Trageinrichtung und die zweite Trageinrichtung müssen nicht unmittelbar aneinander anliegen; es können auch geeignete Zwischenelemente oder Abstandelemente Verwendung finden.

Die Ausricht- und Fixiereinrichtung kann weiterhin geometrische Strukturen (Vorsprünge, Ausnehmungen oder dgl.) umfassen, die für eine (Vor-)Positierung der Kontaktträger bzw. der Trageinrichtungen zueinander sorgen, damit die Bildung der Kontaktelementpaare problemlos erfolgt.

Vorteilhafter Weise ist beim Bilden der Kontaktelementpaare der zweite Kontaktträger durch den ersten Kontaktträger gegen die Federkraft verschiebbar, oder umgekehrt. Wenn dies zudem unmittelbar, das heißt ohne Zwischenschaltung weiterer Elemente geschieht, ergibt sich eine besondere einfache Ausgestaltung der Anordnung.

Vorzugsweise ist die erzeugbare Federkraft größer als eine Summe der durch die als Federkontakte ausgebildeten ersten oder zweiten Kontaktelemente, um jederzeit für eine sichere Kontaktierung im Betrieb sorgen zu können.

Es können jeweils 18 erste und zweite Kontaktelemente vorgesehen sein, die jeweils in einer etwa hexagonalen Konfiguration angeordnet sein können, ohne dass die Erfindung hierauf beschränkt wäre. Vorzugsweise können drei Reihen mit jeweils sechs Kontaktelementen Verwendung finden, wobei die mittlere Reihe in zwei Gruppen zu je drei Kontaktelementen unterteilt sein kann, welche Gruppen voneinander so weit beabstandet sind, dass sich die genannte hexagonale Anordnung ergibt. Einige der ersten (und zweiten) Kontaktelemente können im Rahmen einer entsprechenden Ausgestaltung der Erfindung eine besondere (Zusatz-)Funktion erfüllen. Es kann sich hierbei um die jeweils äußersten Kontaktelemente der genannten Gruppen handeln. Es kann vorgesehen sein, dass diese beiden Kontaktelemente das betreffende Gegen-Kontaktelement an dem anderen Kontaktträger elektrisch leitend kontaktieren müssen, damit das Abluftbehandlungssystem einen ordnungsgemäßen Betriebszustand einnimmt bzw. aktiviert werden kann. Den beiden Kontaktelementen und den betreffenden Gegen-Kontaktelementen kommt also eine Sicherheitsfunktion zu.

Zumindest ein Material des zweiten Kontaktträgers und/oder ein Material der zweiten Kontaktelemente sollte spülmaschinenfest ausgebildet sein, wenn diese - ggf. zusammen mit der betreffenden Trageinrichtung - entsprechend gereinigt werden sollen. Die betreffenden Kontaktelemente können dabei in einer wasserdichten und temperaturfesten (bis etwa +70 °C oder mehr) Masse (Vergussmasse) eingebettet sein. Vorzugsweise ist das Material zumindest des zweiten Kontaktträgers nicht hygroskopisch, wenn dieser an dem zu reinigenden Teil des Abluftbehandlungssystem angeordnet ist.

Vorteilhafter Weise gilt dies bei entsprechender Weiterbildung auch für eine Materialpaarung des Materials des zweiten Kontaktträgers und des Materials der zweiten Kontaktelemente, welche Materialien vorzugsweise ein ähnliches Ausdehnungsverhalten mit der Temperatur aufweisen, damit es nicht zu Spaltbildungen und sonstigen Beschädigungen kommt.

Die beschriebene elektrische Kontakteinrichtung lässt sich somit auch als ein kontaktsicheres, elektrisches Andocksystem mit dynamischen Lage- und Toleranzausgleich und Abdeckfunktion zur sicheren und medienbeständigen elektrischen Verbindung zwischen zwei oder mehr Bauteilen beschreiben, welches sich für den Einsatz in lufttragenden Medien, insbesondere in öl- und fettbelasteten Abluftsystemen, eignet.

Nachfolgend sei auf bestimmte vorteilhafte Eigenschaften gemäß entsprechenden Weiterbildungen der Erfindung nochmals detailliert eingegangen:
Bei Elektrogeräten mit auswechselbaren Elektrobaugruppen - insbesondere den exemplarisch bereits erwähnten UV-Einheiten - wurden zum Elektroanschluss der einzelnen Wechseleinheiten nach dem Stand der Technik Kabel mit Steckverbinder-Systemen verwendet (vgl. die bereits eingangs erwähnte DE 601 33 086 T2).

Während sich diese traditionelle Lösung bei einfachen Geräten weitgehend bewährt hat, erfordert sie zum Koppeln und Entkoppeln der einzelnen Module immer mehrere Schrittfolgen. Wenn in einem Anwendungsbeispiel das entkoppelte Modul zur Wartung oder Reinigung in eine Reinigungslösung verbracht wird und der sichere Schutz der Kontaktelemente (Pins) im Steckverbinder vergessen wird - zum Beispiel durch Unterlassen des Deckelschließens und damit verbundenen Eintritt des Reinigungsmediums in den Steckverbinder -, besteht die Gefahr des Systemausfalles durch Kurzschluss bei Kontakt mit dem Reinigungsmedium. Gerade in stark frequentierten Betriebsstätten, wie Großküchen, wo es auf ein schnelles und sicheres Auswechseln der Systemkomponenten ankommt, ist diese Gefahr immer gegeben.

Die vorgestellte Erfindung vermeidet in der Anwendung Systemausfälle aufgrund von solchen Bedienfehlern. Die elektrisch zu entkoppelnden Elektrobauteile können dem Gesamtsystem entnommen und wieder zugeführt werden, ohne dass Steckerdeckel oder dgl. geöffnet und geschlossen werden müssen. Diese Möglichkeit ist besonders wichtig für den Einsatz von Geräten mit kompakten Funktionen unter besonders widrigen Umfeld-Bedingungen. So einen Kabel- und Steckverbinder freien Elektrokontakt stellt die beschriebene elektrische Kontakteinrichtung (Kontaktblock) dar.

Es können insbesondere folgende Anforderungen an den Einsatz des Kontaktblockes bestehen, die im Rahmen entsprechender Weiterbildungen der Erfindung erfüllbar sind:
- Schutz der Kontakte vor mechanischen Beschädigungen bei der Verwendung; sichere Positionierung der beiden Blockhälften (Kontaktträger) zueinander, einschließlich Vorpositionierung z.B. mittels Führungsschienen und Endanschlägen, Feinpositionierung (bis auf +/- 0,25 mm) durch z.B. Führungsbolzen- und Bohrungen sowie automatische Positionssicherung mit mechanischer Lagearretierung;
- sichere elektrische Kontaktierung, z.B. mit einem Kontaktdruck von mindestens 1,0 N und 3 A Dauerstrom bei einem Federweg der Federkontakte von mindestens 1,2 mm;
- chemisch stabile Kontaktflächen durch z.B. vergoldete Oberflächenbeschichtung; Einsatz von Edelstahl-Kontakt-Druckfedern;
- sichere, mechanische Abschottung von der Einsatzumgebung;
- modularer Aufbau des Kontaktblockes für eine stabile Funktion und für die Sicherung der einfachen Montage sowie anfallender Reparaturen, insbesondere durch Vorsehen vakuumstabiler (federnder) Kontaktelemente und/oder - falls gewünscht bzw. erforderlich - durch leichte und demontagefreie Auswechselbarkeit von einzelnen, funktionsgestörten Kontaktelementen.

Durch Anwendung des vorgeschlagenen Kontaktblockes können sich - je nach Ausgestaltung - folgende Vorteile ergeben:
- einfacher und schneller Ein- und Ausbau der Elektro-Baugruppen, sicherer Anschluss der Wechselmodule mit dem Elektrogerät und auch untereinander;
- schnelle und effektive Durchführung der Reparaturen an den Baugruppen.
- der Austausch der Wechselbaugruppen auch durch ungeschultes Personal möglich;
- einfache Signalbündelung durch Leiterplattenanwendung am Kontaktblock.

Weitere Eigenschaften und Vorteile der Erfindung ergeben sich aus der nachfolgenden Beschreibung von Ausführungsbeispielen anhand der Zeichnung.
- Figur 1: zeigt eine perspektivische Gesamtansicht eines erfindungsgemäßen Abluftbehandlungssystems mit einer elektrischen Kontakteinrichtung;
- Figur 2: zeigt eine Ausschnittsvergrößerung des Ausschnitts X in Figur 1;
- Figur 3: zeigt das Abluftbehandlungssystem gemäß Figur 1 in einer ähnlichen Darstellung;
- Figur 4: zeigt eine Ausschnittsvergrößerung gemäß Bezugszeichen Y in Figur 3;
- Figur 5: zeigt eine andere Ansicht des erfindungsgemäßen Abluftbehandlungssystems gemäß Figur 1;
- Figur 6: zeigt eine Ausschnittsvergrößerung gemäß Bezugszeichen Z in Figur 5;
- Figur 7: zeigt einen Schnitt durch eine elektrische Kontakteinrichtung in ihrem geöffneten Zustand;
- Figur 8: zeigt einen Schnitt durch die Kontakteinrichtung gemäß Figur 7 in ihrem geschlossenen Zustand;
- Figur 9: zeigt eine weitere Detailansicht des erfindungsgemäßen Abluftbehandlungssystems im Bereich der elektrischen Kontakteinrichtung in ihrem offenen Zustand;
- Figur 10: zeigt eine weitere Ansicht des erfindungsgemäßen Abluftbehandlungssystems mit der elektrischen Kontakteinrichtung in ihrem geschlossenen Zustand; und
- Figur 11: zeigt noch eine andere Ansicht des erfindungsgemäßen Abluftbehandlungssystems mit der elektrischen Kontakteinrichtung in ihrem geschlossenen Zustand.

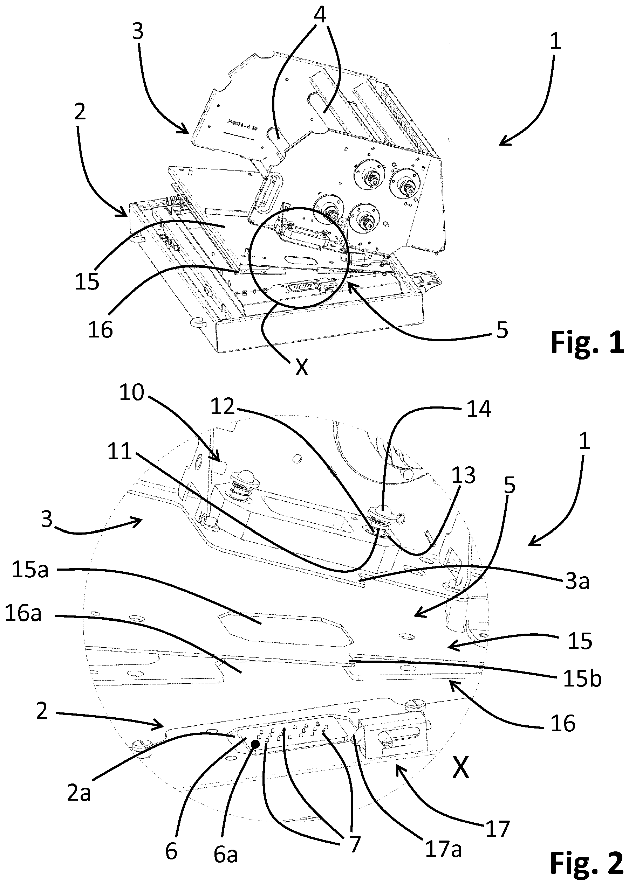

In Figur 1 ist bei Bezugszeichen 1 ein erfindungsgemäßes Abluftbehandlungssystem in einer perspektivischen Gesamtansicht in einem teilweise demontierten Zustand gezeigt. Das Abluftbehandlungssystem 1 eignet sich insbesondere - jedoch ohne Beschränkung - für eine Verwendung in Großküchen. Es umfasst wenigstens ein sogenanntes Trageteil 2 und wenigstens ein an dem Trageteil 2 angeordnetes oder anordenbares, abnehmbares Gehäuseteil 3, welches Gehäuseteil 3 im Betrieb des Abluftbehandlungssystems 1 von Abluft durchströmt oder durchströmbar ist. Zu diesem Zweck ist das gezeigte Abluftbehandlungssystem 1 mit einem (Saug-)Gebläse oder dergleichen und gegebenenfalls weiteren Abluftleitungselementen verbunden, welche in Figur 1 nicht dargestellt sind. In dem Gehäuseteil 3 ist eine Anzahl von UV-Einheiten angeordnet, von denen in Figur 1 aus Gründen der Übersichtlichkeit nur einige mit dem Bezugszeichen 4 explizit bezeichnet sind. Die UV-Einheiten 4 dienen in an sich bekannter Weise dazu, die Abluft im Bereich des Gehäuseteils 3 mit elektromagnetischer Strahlung im UV-Bereich zu beaufschlagen. Dies ist dem Fachmann an sich bekannt. Zumindest das Gehäuseteil 3 lässt sich in der in Figur 1 angedeuteten Weise von dem Trageteil 2 trennen, um es als ganzes, das heißt mitsamt den UV-Einheiten 4 in einfacher Weise reinigen zu können, vorzugsweise in einer Küchenspülmaschine.

In dem in Figur 1 mit dem Bezugszeichen X gekennzeichneten Bereich weist das Abluftbehandlungssystem 1 eine elektrische Kontakteinrichtung 5 auf, auf deren genaue Ausgestaltung nachfolgend genauer eingegangen wird. Die Kontakteinrichtung 5 dient insbesondere dazu, die UV-Einheit 4 in dem verbundenen (Betriebs-)Zustand von Trageteil 2 und Gehäuseteil 3 mit elektrischer Energie und gegebenenfalls Steuersignalen oder dergleichen zu versorgen.

Figur 2 zeigt einen Detailausschnitt gemäß Bezugszeichen X in Figur 1. Hier und in den nachfolgenden Figuren bezeichnen gleiche Bezugszeichen gleiche oder gleichwirkende Elemente.

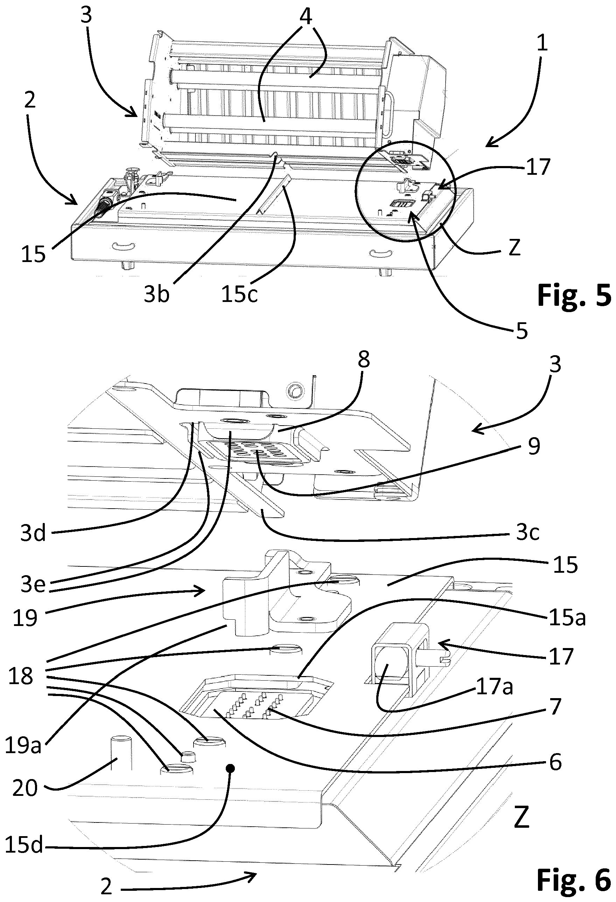

Gemäß Figur 2 umfasst die bereits erwähnte Kontakteinrichtung 5 an dem Trageteil 2 einen ersten Kontaktträger 6, der eine erste Anzahl erster Kontaktelemente aufweist, von denen in Figur 2 aus Gründen der Übersichtlichkeit nur einige wenige mit dem Bezugszeichen 7 explizit bezeichnet sind. Des Weiteren umfasst die Kontakteinrichtung 5 an dem Gehäuseteil 3 einen zweiten Kontaktträger 8, der eine zweite Anzahl zweiter Kontaktelemente aufweist, die aufgrund der gewählten Darstellung in Figur 2 jedoch nicht zu erkennen sind. In Figur 6 und in weiteren Figuren sind diese zweiten Kontaktelemente mit dem Bezugszeichen 9 bezeichnet. Vorliegend entspricht die erste Anzahl der zweiten Anzahl, ohne dass die Erfindung hierauf beschränkt wäre. Die Kontakteinrichtung 5 umfasst weiterhin eine Federkrafteinrichtung 10, die gemäß dem gezeigten Ausführungsbeispiel aus zwei Schraubenfedern 11 gebildet ist, die in der gezeigten Art und Weise um jeweils einen an dem Gehäuseteil 3 vorgesehenen Bolzen oder Stift 12 herum angeordnet sind, welche Bolzen oder Stifte 12 sich durch entsprechende Durchbrüche 13 in dem ersten Kontaktträger 8 hindurch erstreckt. In einem aus dem Kontaktträger 8 herausragenden freien Ende der Stifte oder Bolzen 12 sind Widerlager 14 für die Federn 11 vorgesehen, wie gezeigt. Der Kontaktträger 8 ist bezüglich einer Bewegung längs der Stifte oder Bolzen 12 gegenüber dem Gehäuseteil 3 beweglich gelagert.

Bei dem dargestellten Ausführungsbeispiel (vergleiche beispielsweise Figur 6) entspricht die Anzahl und die Anordnung der ersten Kontaktelemente 7 gerade der Anzahl zweiter Kontaktelemente 9, was jedoch nicht zwingend der Fall sein muss. Dabei sind die ersten Kontaktelemente 7 und die zweiten Kontaktelemente 9 derart angeordnet, dass sie gemeinsam mit jeweils einem Kontaktelement an dem anderen Kontaktträger 6, 8 paarweise in elektrisch leitenden Kontakt gebracht oder bringbar sind, um in einem verbundenen Zustand der Kontakteinrichtung 5 entsprechende Kontaktelementpaare zu bilden.

Bei dem gezeigten Ausführungsbeispiel sind die erste Kontaktelemente 7 als Federkontakte ausgebildet, deren in Figur 2 erkennbare Spitzen zumindest in dem gezeigten unverbundenen Zustand der Kontakteinrichtung 5 um ein gewisses Übermaß (hier nicht bezeichnet) über eine Oberfläche 6a des betreffenden Kontaktträgers 6 hinausragen. Grundsätzlich liegt es im Rahmen der Erfindung, zusätzlich oder alternativ auch die zweiten Kontaktelemente 9 als Federkontakte auszubilden. Die genannte Oberfläche 6a des ersten Kontaktträgers 6 ist zumindest in dem bereits angesprochenen, verbundenen Zustand der Kontakteinrichtung 5 (in Figur 2 nicht gezeigt) dem anderen Kontaktträger 8 zugewandt. Durch die Federkrafteinrichtung 10 ist eine Federkraft auf den zweiten Kontaktträger 8 in Richtung auf den ersten Kontaktträger 6 bewirkbar, wenn in dem verbundenen Zustand der Kontakteinrichtung 5 die genannten Kontaktelementpaare gebildet sind. Hierauf wird weiter unten noch ausführlich eingegangen.

Weiter ist insbesondere Figur 2 noch Folgendes zu entnehmen: Der erste Kontaktträger 6 ist innerhalb einer Vertiefung 2a des Trageteils 2 angeordnet. Weiterhin befinden sich zwischen dem Gehäuseteil 3 und dem Trageteil 2 noch zwei weitere flache Abstandselemente oder Rahmenteile, die in Figur 2 mit dem Bezugszeichen 15 bzw. 16 bezeichnet sind. Das Abstandselement 15 weist einen Durchbruch 15a auf, dessen Form und Anordnung im Wesentlichen mit der Vertiefung 2a korrespondiert. Das Abstandselement 16 umfasst eine entsprechende Ausnehmung 16a, die ebenfalls so angeordnet und dimensioniert ist, dass durch die genannten Durchbrüche bzw. Ausnehmungen 15a, 16a die angesprochene Bildung von Kontaktelementpaaren zwischen dem ersten Kontaktträger 6 und dem zweiten Kontaktträger 8 möglich ist.

Des Weiteren umfasst die in Figur 2 gezeigte Anordnung bei Bezugszeichen 17 noch eine Fixiereinrichtung in Form einer Rast- oder Schnappvorrichtung, welche dazu ausgebildet ist, die Kontakteinrichtung 5 in ihrem verbundenen Zustand zu halten, worauf weiter unten noch genauer eingegangen wird. Wie sich jedoch bereits der Figur 2 entnehmen lässt, umfasst die Fixiereinrichtung 17 ein abgeschrägtes Verriegelungselement (Sperrklinke) 17a, welches selbsttätig (beispielsweise durch Vorsehen eines geeigneten Federmittels) die in Figur 2 gezeigte Stellung einnimmt. Wenn nun das Gehäuseteil 3 sowie die Abstandselemente 15, 16 aus der Darstellung gemäß Figur 2 nach unten bewegt und auf dem Trageteil 2 abgelegt werden, tritt Element 17a mit einem an dem Gehäuseteil 3 bzw. an dem Abstandselement 15 vorgesehenen Vorsprung 3a bzw. 15b in Wechselwirkung, welche das Element 17a entgegen der exemplarisch erwähnten Federkraft verschieben. Anschließend bewegt sich das Element 17a wieder in seine in Figur 2 gezeigte (Verriegelungs-)Stellung, sodass das Gehäuseteil 3 und die Abstandselemente 15, 16 in ihrer Anlage an dem Trageteil 2 gehalten sind. Hierauf wird weiter unten ebenfalls noch genauer eingegangen. Selbstverständlich ist die Erfindung nicht auf die beispielhaft beschriebene Ausgestaltung der Fixiereinrichtung 17 beschränkt.

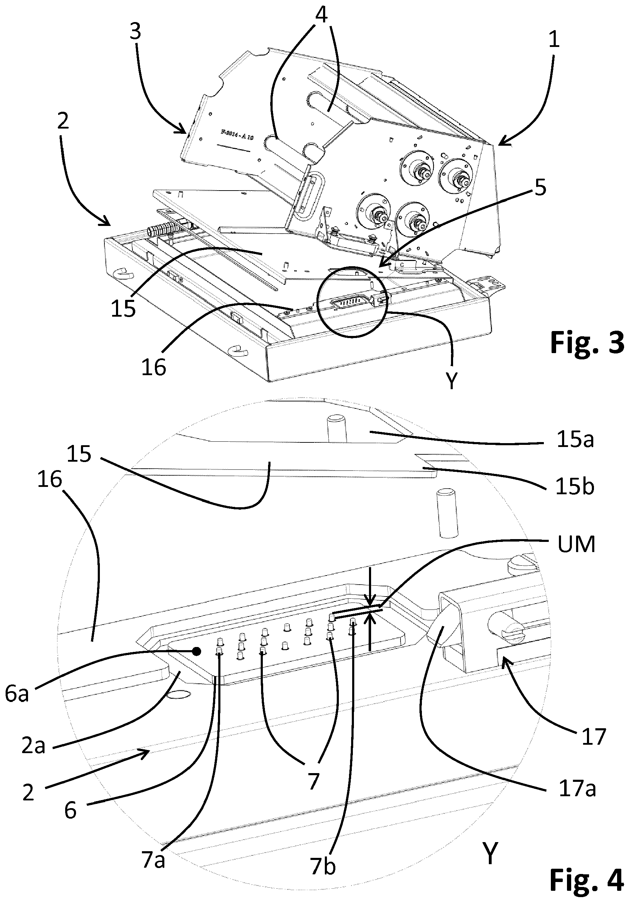

Figur 3 zeigt nochmals das bereits auf Figur 1 bekannte Abluftbehandlungssystem 1, sodass hierauf zunächst nicht weiter einzugehen ist. Zu erwähnen ist lediglich, dass, anders als in Figur 1, bei dem Zustand gemäß Figur 3 das Abstandselement 16 in seinem an dem Trageteil 2 anliegenden Zustand gezeigt ist.

Figur 4 zeigt eine Ausschnittsvergrößerung gemäß Bezugszeichen Y in Figur 3. Diese Ausschnittvergrößerung illustriert noch einmal besonders anschaulich die Ausbildung des ersten Kontaktträgers 6 mit den als Federkontakten ausgebildeten ersten Kontaktelementen 7, die jeweils um ein Übermaß UM über die Oberfläche 6a des Kontaktträgers 6 hinausragen. Das Abstandselement 16 liegt an dem Trageteil 2 an, wie bereits mit Blick auf die Figur 3 erwähnt wurde.

Einige der ersten Kontaktelemente 7 können im Rahmen einer entsprechenden Ausgestaltung der Erfindung eine besondere (Zusatz-)Funktion erfüllen. Es handelt sich hierbei um die in Figur 4 mit den Bezugszeichen 7a und 7b bezeichneten äußersten Kontaktelemente der insgesamt etwa hexagonalen Anordnung der ersten Kontaktelemente 7. Es kann vorgesehen sein, dass diese beiden Kontaktelemente 7a, 7b das betreffende Gegen-Kontaktelement an dem zweiten Kontaktträger (in Figur 4 jeweils nicht gezeigt) elektrisch leitend kontaktieren müssen, damit das Abluftbehandlungssystem einen ordnungsgemäßen Betriebszustand einnimmt bzw. aktiviert werden kann. Den Kontaktelementen 7a, 7b und den betreffenden Gegen-Kontaktelementen kommt also eine Sicherheitsfunktion zu.

Figur 5 zeigt eine etwas andere perspektivische Darstellung des Abluftbehandlungssystems 1. Hier liegt nun auch das Abstandselement 15 an dem Trageteil 2 bzw. dem (nicht mehr erkennbaren) Abstandselement 16 an. Es wird durch die Fixiereinrichtung 17 in der Stellung gemäß Figur 5 gehalten und gesichert. Weiterhin ist der Figur 5 noch entnehmbar, dass an dem Gehäuseteil 3 eine Reihe von im Querschnitt etwa dreieckförmigen, fluchtenden Aussparungen 3b vorgesehen sind, von denen aus Gründen aus der Übersichtlichkeit nur eine explizit bezeichnet ist. Die Form und Anordnung dieser Aussparungen 3b ist komplementär zu einer vorspringenden Struktur 15c an der Oberseite des Abstandselements 15. Das Zusammenwirken der genannten Strukturen 3b, 15c soll eine Zentrierung des Gehäuseteils 3 bezüglich der restlichen Anordnung, insbesondere des Tragteils 2, bewirken, um auf diese Weise auch das Schließen der Kontakteinrichtung 5 zu erleichtern.

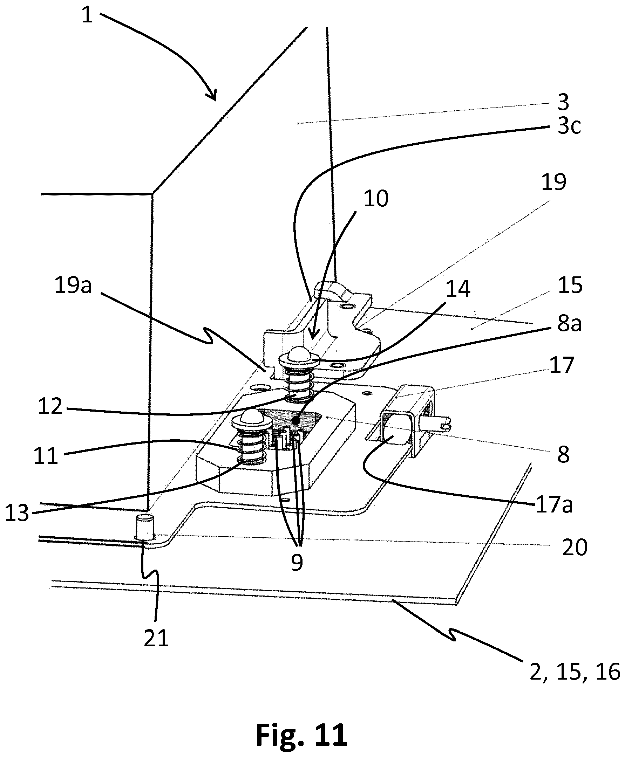

In Figur 6 ist eine Ausschnittsvergrößerung bei Bezugszeichen Z in Figur 5 gezeigt. Es sei nachfolgend nur auf ausgewählte Einzelmerkmale gemäß Figur 6 näher eingegangen:
Wie auch bereits der Darstellung in Figur 1 andeutungsweise zu entnehmen war, weist das Trageteil 2 an seiner Oberseite eine Reihe von vorspringenden Strukturen in Form von Bolzen oder Stiften auf, die in Figur 6 größtenteils verallgemeinernd mit dem Bezugszeichen 18 bezeichnet sind. Diese Strukturen 18 korrespondieren mit entsprechenden Durchbrüchen (nicht bezeichnet) in den Abstandselementen 15, 16, sodass sie gemäß der Darstellung in Figur 6 nach Anordnung der Abstandselemente 15, 16 auf dem Trageteil 2 nach oben aus dem Abstandselement 15 heraus stehen. Auf diese Weise sorgen die Strukturen 18 für eine Ausrichtung der genannten Elemente 2, 15, 16 zueinander und können darüber hinaus weiterhin für eine entsprechende Ausrichtung des Gehäuseteils 3 genutzt werden. Des Weiteren ist an der Oberseite des Abstandselements 15 gemäß Figur 6 eine weitere Ausrichtstruktur 19 und ein weiterer Ausrichtbolzen 20 angeordnet, welche zum Ausrichten des Gehäuseteils 3 dienen. Hierauf wird weiter unten noch genauer eingegangen. Entsprechende Strukturen (19, 20) finden sich auch auf der (nicht gezeigten) anderen Seite der Anordnung. Es sei allerdings bereits jetzt darauf hingewiesen, dass die Ausrichtstruktur 19 eine der Oberseite 15d des Abstandselements 15 benachbarte Ausnehmung 19a aufweist, welche dazu ausgebildet ist, eine entsprechende Abkantung 3c des Gehäuseteils aufzunehmen, um so ein Hochschwenken oder Hochklappen des Gehäuseteils 3 winkelmäßig zu beschränken. Hierauf wird weiter unten insbesondere anhand von Figur 11 noch genauer eingegangen.

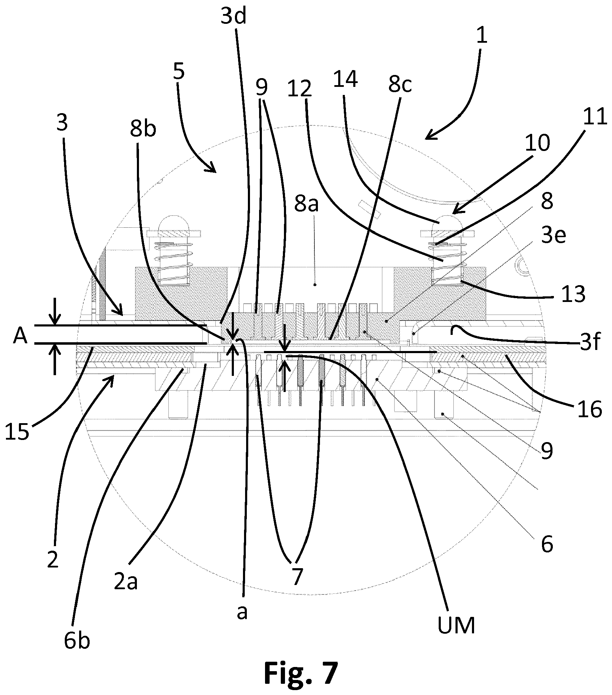

In Figur 7 ist die bereits mehrfach erwähnte elektrische Kontakteinrichtung 5 im Schnitt dargestellt. Gut erkennbar ist hier wiederum das Übermaß UM der als Federkontakte ausgebildeten ersten Kontaktelemente 7 bezüglich des ersten Kontaktträgers 6. Letzterer ist vorzugsweise in einem (nicht-faserverstärkten) Kunststoff, beispielsweise PEEK, ausgebildet, und umschließt die Kontaktelemente 7 seitlich in der dargestellten Weise. Die Kontaktelemente 9 in dem zweiten Kontaktträger 8 sind an ihrer den Kontaktelementen 7 zugewandten Stirnseitenfläche verbreitert ausgebildet, um eine sichere Kontaktierung bzw. eine sichere Bildung der erwähnten Kontaktelementpaare zu gewährleisten. Auch der Kontaktträger 8 ist vorzugsweise in einem elektrisch isolierenden Kunststoffmaterial, beispielsweise PEEK, ausgebildet. Die Kontaktträger 6, 8 müssen jedoch nicht in ein und demselben Material ausgebildet sein. Da der Kontaktträger 8 mitsamt den Kontaktelementen 9 zusammen mit dem gesamten Gehäuseteil 3 in einer Spülmaschine gereinigt werden soll, ist eine entsprechend wasser- und temperaturbeständige Ausführung des Kontaktträgers 8 und der Kontaktelemente 9 sinnvoll und bevorzugt. Der Kontaktträger 8 kann die in Figur 7 gezeigte zentrale Ausnehmung 8a aufweisen, die mit einer wasser- und temperaturfesten Vergußmasse gefüllt ist, welche die Kontaktelemente 9 nach oben abdichtet, und dabei gleichzeitig noch für eine elektrische Kontaktierung der Kontaktelemente 9 sorgt bzw. eine solche ermöglicht.

Wie bereits der Figur 6 zu entnehmen war, besitzt das Gehäuseteil 3 einen Durchbruch 3d, in dem der Kontaktträger 8 teilweise aufgenommen ist, wie dargestellt. In Randbereichen dieses Durchbruches 3d weist das Gehäuseteil 3 Abkantungen 3e (vgl. Figur 6) auf, die etwa derart weit gegenüber einer Unterseite 3f des Gehäuseteils 3 vorspringen, wie auch der Kontaktträger 8 im Bereich des Durchbruchs 3d gegenüber dem Gehäuseteil 3 hervorsteht (Maß A, vgl. Figur 7). In diesem Zusammenhang ist zu erwähnen, dass der Kontaktträger 8 bei dem gezeigten Ausführungsbeispiel einen umlaufenden Rand 8b aufweist, der um ein Maß a gegenüber einer sonstigen Oberfläche 8c des Kontaktträgers 8, in welcher die Kontaktelemente 9 flächenbündig angeordnet sind, hervorsteht. Dabei gilt UM > a. Der Überstand der Abkantungen 3e gegenüber der Unterseite 3f des Gehäuseteils 3 ist in Figur 7 mit dem Bezugszeichen A bezeichnet. Die Abkantungen 3f schützen den Kontaktträger 8 und die Kontaktelemente 9 vor mechanischer Schadeinwirkung - insbesondere, wenn das Gehäuseteil 3 von dem Trageteil 2 bzw. den Abstandselementen 15, 16 getrennt ist. Zusätzlich oder alternativ kann (auch) der andere Kontaktträger 6 einen solchen umlaufenden Rand (nicht gezeigt) aufweisen.

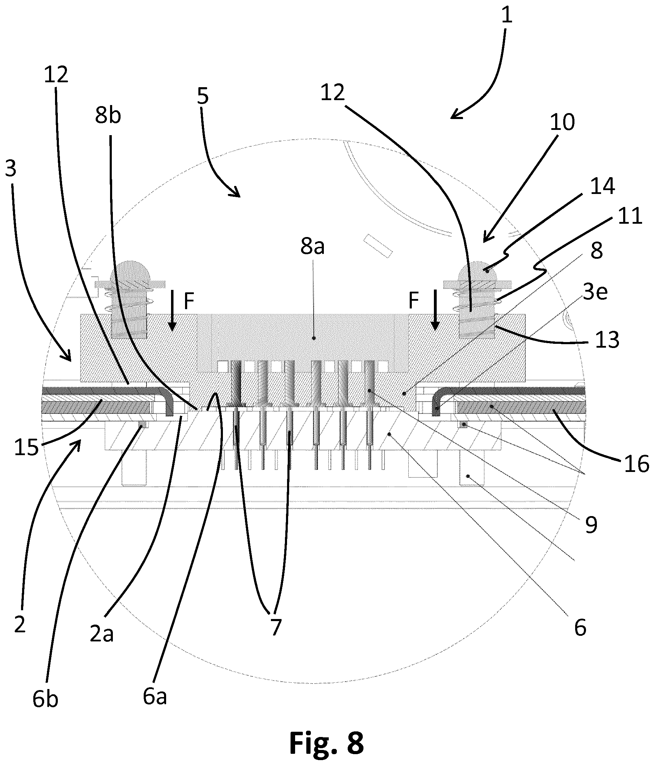

Der Kontaktträger 8 ist gegenüber dem restlichen Gehäuseteil 3 beweglich angeordnet, und zwar längs einer Erstreckungsrichtung der bereits erwähnten Bolzen 12 entgegen einer durch die Feder 11 vermittelten Federkraft, wenn die Feder 11 mit Element 14 in Kontakt tritt. Dieser Sachverhalt wurde bereits erwähnt und ist in der nachfolgenden Figur 8 genauer dargestellt.

Figur 8 zeigt im Wesentlichen die Anordnung gemäß Figur 7, jedoch in dem verbundenen oder geschlossenen Zustand der Kontakteinrichtung 5. Dieser Zustand ist dann erreicht, wenn das Gehäuseteil 3 mit seinem Vorsprung 3a hinter dem Element 17a der Fixiereinrichtung 17 (vergleiche Figur 2) niedergehalten ist. Der Kontaktträger 8 liegt dann mit seinem vorspringenden Rand 8b an der Oberfläche 6a des Kontaktträgers 6 an, sodass die Kontaktelemente bzw. Federkontakte 7 die Kontaktelemente 9 zwecks Bildung der genannten Kontaktelementpaare berühren. Da das Übermaß UM der Kontaktelemente 7 bezüglich der Oberfläche 6a des Kontaktträgers 6 größer ist als eine Höhe des Vorsprungs 8b (UM > a) kommt es zur Bildung der genannten Kontaktelementpaare bei gleichzeitiger seitlich umlaufender Abdichtung. Gleichzeitig wird der Kontaktträger 8, geführt durch die Bolzen 12, nach oben verschoben, sodass die Federn 11 mit den Elementen 14 in Wechselwirkung treten, was die in Figur 8 mit Bezugszeichen F bezeichnete Federkraft zur Folge hat, die auf den Kontaktträger 8 wirkt und diesen sicher und definiert entgegen einer umgekehrt orientierten Federkraft, die durch die Gesamtheit der Kontaktelemente 7 vermittelt wird, in Anlage an den Kontaktträger 6 zwingt, sodass die bestimmungsgemäße Ausbildung der bereits mehrfach erwähnten Kontaktelementpaare sicher gewährleistet ist. Gemäß der Darstellung in Figur 8 führt dies zu einem geringfügigen Abheben des Kontaktträgers 8 von dem Gehäuseteil 3.

Zu entnehmen ist insbesondere der Figur 8 ansonsten noch eine an der Oberseite des Kontaktträgers 6 angeordnete, umlaufende Dichtung 6b, welche den Bereich der bereits mehrfach angesprochenen Vertiefung 2a seitlich gegenüber dem restlichen Trageteil 2a abdichtet.

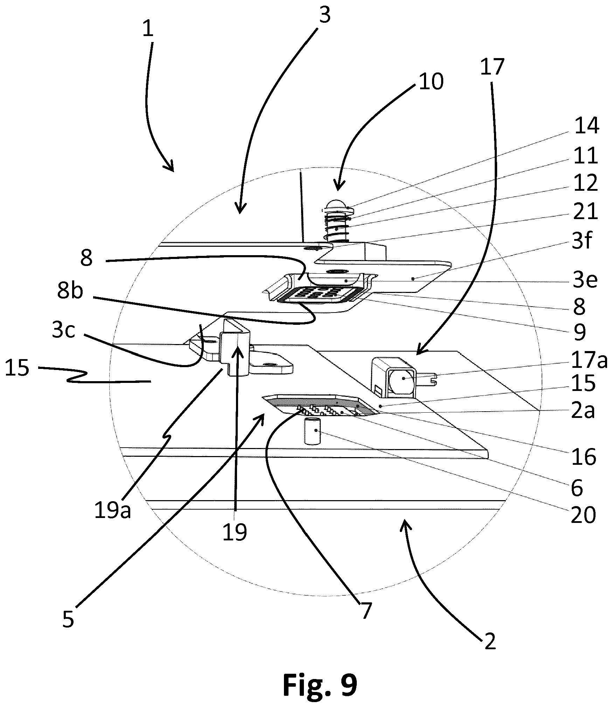

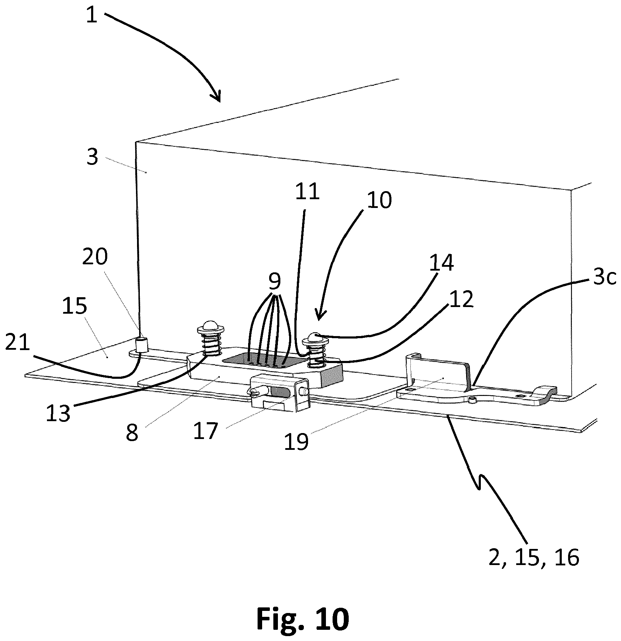

Figur 9 zeigt eine weitere, perspektivische Detailansicht des Abluftbehandlungssystems 1 im Bereich der Kontakteinrichtung 5 in ihrem geöffneten Zustand. Dort ist auch ein Durchbruch 21 an dem Gehäuseteil 3 erkennbar, der zu Ausrichtzwecken mit dem weiter oben erwähnten Ausrichtbolzen 20 zusammenwirkt (vgl. Figur 10).

Der Figur 10 ist insbesondere noch zu entnehmen, wie das Gehäuseteil 3 mit der anhand von Figur 6 eingeführten Ausrichtstruktur 19 bzw. dem Ausrichtbolzen 20 zusammenwirkt. Speziell hintergreift bzw. untergreift das Gehäuseteil 3 mit der bereits erwähnten Abwinkelung 3c die Ausrichtstruktur 19 im Bereich der Ausnehmung 19a. Des Weiteren ist erkennbar, wie der an dem Gehäuseteil 3 vorhandene Durchbruch 21 mit dem Ausrichtbolzen 20 zusammenwirkt.

Dieser Sachverhalt ist auch in Figur 11 nochmals aus anderem Blickwinkel dargestellt.

Es folgt abschließend eine kurze Beschreibung des Positionier-und Kontaktierungsvorganges:
Das Wechselmodul (vorliegend: das Gehäuseteil 3) wird zwischen den beiden seitlich angeordneten Führungs- und Endanschlagsblocks bzw. Ausrichtstrukturen 19 eingelegt und bis zu dem in Figur 10 oder 11 gezeigten Anschlag gerückt (Vorpositionierung). In dieser Position stehen Führungsbolzen und Positionsbohrung in Form der betreffenden Elemente 20 und 21 genau übereinander. Durch eine vorzugsweise konische Form des Führungsbolzens 20 ist die Feinpositionierung nach dem Herunterdrücken des Wechselmoduls (Gehäuseteil 3) gesichert. Zur gleichen Zeit rastet die Lagearretierung gemäß Bezugszeichen 17, 17a ein. Die Kontaktelemente 7 und 9 liegen dann vorzugsweise +/-0,25 mm genau aufeinander.

Durch das Herunterdrücken des Wechselmoduls (Gehäuseteil 3) liegt der Kontaktträger 8 auf dem Kontaktträger 6 auf und hebt sich nach dem Einrasten der Lagearretierung gegen die Druckfedern 11 etwas ab. Die beiden Druckfedern 11 sorgen für einen stetigen Kontakt der beiden Trägerelemente (Kontaktträger) 6, 8. Somit ist eine mechanische Abschottung der Kontakte von den Umgebungseinflüssen gegeben.

Die Entnahme des Wechselmoduls ist nach der Entriegelung der Lagearretierung in umgekehrter Reihenfolge möglich. Das Wechselmodul (Gehäuseteil 3) kann dann insbesondere in einer Spülmaschine gereinigt werden.

## Patentansprüche

1. Abluftbehandlungssystem (1), insbesondere für Großküchen, mit wenigstens einem Trageteil (2) und mit wenigstens einem an dem Trageteil (2) angeordneten oder anordbaren, abnehmbaren Gehäuseteil (3), welches Gehäuseteil (3) im Betrieb des Abluftbehandlungssystems (1) von Abluft durchströmt oder durchströmbar ist und in welchem Gehäuseteil (3) wenigstens eine UV-Einheit (4) angeordnet ist, durch die die Abluft mit elektromagnetischer Strahlung im UV-Bereich beaufschlagbar ist, wobei an dem Trageteil (2) und an dem Gehäuseteil (3) gemeinsam wenigstens eine elektrische Kontakteinrichtung (5) vorgesehen ist,
**dadurch gekennzeichnet, dass**
die elektrische Kontakteinrichtung (5) umfasst:
einen ersten Kontaktträger (6) mit einer ersten Anzahl erster Kontaktelemente (7);
einen zweiten Kontaktträger (8) mit einer zweiten Anzahl zweiter Kontaktelemente (9); und
eine Federkrafteinrichtung (10), die zum Einwirken auf den zweiten Kontaktträger (8) ausgebildet ist;
wobei wenigstens eine Unteranzahl der ersten Kontaktelemente (7) oder der zweiten Kontaktelemente (9) derart angeordnet ist, dass sie gemeinsam mit jeweils einem Kontaktelement an dem anderen Kontaktträger paarweise in elektrisch leitenden Kontakt gebracht oder bringbar sind, um in einem verbundenen Zustand der Kontakteinrichtung Kontaktelementpaare zu bilden;
wobei die ersten Kontaktelemente (7) oder die zweiten Kontaktelemente (9) als Federkontakte ausgebildet sind, deren Spitzen zumindest in einem unverbundenen Zustand der Kontakteinrichtung (5) um ein Übermaß (UM) über eine Oberfläche (6a) des betreffenden Kontaktträgers (6) hinausragen, welche Oberfläche (6a) zumindest in dem verbundenen Zustand dem anderen Kontaktträger (8) zugewandt ist;
wobei durch die Federkrafteinrichtung (10) eine Federkraft (F) auf den zweiten Kontaktträger (8) in Richtung auf den ersten Kontaktträger (6) bewirkt oder bewirkbar ist, wenn die genannten Kontaktelementpaare gebildet sind;
wobei der erste Kontaktträger (6) im Wesentlichen unverschiebbar an einer ersten Trageinrichtung (2) befestigt ist, welche erste Trageinrichtung (2) an dem Trageteil (2) angeordnet ist oder durch dieses Trageteil (2) gebildet ist;
wobei der zweite Kontaktträger (8) an einer zweiten Trageinrichtung (3) befestigt und gegenüber dieser zweiten Trageinrichtung (3) gegen die durch die Federkrafteinrichtung (10) bewirkte oder bewirkbare Federkraft (F) verschiebbar ist, welche zweite Trageinrichtung (3) an dem Gehäuseteil (3) angeordnet ist oder durch dieses Gehäuseteil (3) gebildet ist; und
wobei der verbundene Zustand der Kontakteinrichtung erreicht wird beim Anordnen des Gehäuseteils (3) an dem Trageteil (2).

2. Abluftbehandlungssystem (1) nach Anspruch 1,
**dadurch gekennzeichnet, dass**
der erste Kontaktträger (6) und der zweite Kontaktträger (8) in dem verbundenen Zustand jeweils mit wenigstens einer Teilfläche ihrer jeweiligen Oberflächen (6a, 8c) aneinander anliegen.

3. Abluftbehandlungssystem (1) nach Anspruch 2,
**dadurch gekennzeichnet, dass**
der erste Kontaktträger (6) und/oder der zweite Kontaktträger (8) im Bereich der jeweiligen Teilfläche eine vorspringende Struktur (8b) und/oder ein Dichtelement aufweist.

4. Abluftbehandlungssystem (1) nach Anspruch 3,
**dadurch gekennzeichnet, dass**
die vorspringende Struktur (8b) und/oder das Dichtelement um die jeweiligen ersten (7) und/oder zweiten Kontaktelemente (9) umlaufend ausgebildet ist, vorzugsweise geschlossen.

5. Abluftbehandlungssystem (1) nach Anspruch 3 oder 4,
**dadurch gekennzeichnet, dass**
die vorspringende Struktur (8b) und/oder das Dichtelement in dem verbundenen Zustand eine Höhe (a) gegenüber einer restlichen Oberfläche (8c) des jeweiligen Kontaktträgers (8) aufweist, welche Höhe (a) kleiner ist als das Übermaß (UM) der Federkontakte (7) zuzüglich eines etwaigen Übermaßes der Kontaktelemente (9) des anderen Kontaktträgers (8) gegenüber dessen Oberfläche (8c), welche Oberfläche (8c) zumindest in dem verbundenen Zustand der Oberfläche (6a) des die Federkontakte (7) aufweisenden Kontaktträgers (6) zugewandt ist.

6. Abluftbehandlungssystem (1) nach einem der Ansprüche 1 bis 5,
**dadurch gekennzeichnet, dass**
die nicht als Federkontakte ausgebildeten Kontaktelemente (9) im Wesentlichen flächenbündig mit der Oberfläche (8c) des betreffenden Kontaktträgers (8) in diesem eingebettet sind.

7. Abluftbehandlungssystem (1) nach einem der Ansprüche 1 bis 6,
**dadurch gekennzeichnet, dass**
zumindest in dem verbundenen Zustand die erste Trageinrichtung (2) und die zweite Trageinrichtung (3) bereichsweise flächig aneinander anliegen.

8. Abluftbehandlungssystem (1) nach Anspruch 7,
**dadurch gekennzeichnet, dass**
der erste Kontaktträger (6) oder der zweite Kontaktträger (8) gegenüber einer Fläche der betreffenden Trageinrichtung (2, 3) vorspringend angeordnet ist, während der andere Kontaktträger gegenüber einer Fläche der betreffenden Trageinrichtung zurückversetzt angeordnet ist.

9. Abluftbehandlungssystem (1) nach einem der Ansprüche 1 bis 8,
**dadurch gekennzeichnet, dass**
der erste Kontaktträger (6) und der zweite Kontaktträger (8) im Bereich von Durchbrüchen (2a, 3d) in der jeweiligen Trageinrichtung (2, 3) angeordnet sind.

10. Abluftbehandlungssystem (1) nach Anspruch 8 und Anspruch 9,
**dadurch gekennzeichnet, dass**
an derjenigen Trageinrichtung (3), an welcher Trageinrichtung (3) der betreffende Kontaktträger (8) gegenüber der Fläche der betreffenden Trageinrichtung (3) vorspringend angeordnet ist, in Randbereichen des jeweiligen Durchbruchs (3d) Abkantungen (3e) vorgesehen sind, welche Abkantungen (3e) in eine gleiche Richtung vorspringen wie der betreffende Kontaktträger (8).

11. Abluftbehandlungssystem (1) nach Anspruch 10,
**dadurch gekennzeichnet, dass**
ein Übermaß (A) der Abkantungen (3e) gegenüber der Fläche der betreffenden Trageinrichtung (3) gleich groß oder größer ist als ein Maß des Vorspringens des betreffenden Kontaktträgers (8) gegenüber der Fläche der betreffenden Trageinrichtung (3), und dass
ein Übermaß (A) der Abkantungen (3e) gegenüber der Fläche der betreffenden Trageinrichtung (3) gleich groß oder kleiner ist als ein Maß des Rückversatzes des anderen Kontaktträgers (6) gegenüber der Fläche der anderen Trageinrichtung (2).

12. Abluftbehandlungssystem (1) nach einem der Ansprüche 1 bis 11,
**dadurch gekennzeichnet, dass**
zumindest in dem verbundenen Zustand die erste Trageinrichtung (2) und die zweite Trageinrichtung (3) aneinander lösbar festgelegt sind.

13. Abluftbehandlungssystem (1) nach einem der Ansprüche 1 bis 12,
**dadurch gekennzeichnet, dass**
an der ersten Trageinrichtung (2) und/oder an der zweiten Trageinrichtung (3) wenigstens eine Ausricht- und Fixiereinrichtung (17) vorgesehen ist, vorzugsweise umfassend eine Rast- oder Schnappvorrichtung, welche dazu ausgebildet ist, die Kontakteinrichtung (5) in dem verbundenen Zustand zu halten, höchst vorzugsweise im Wesentlichen selbsttätig beim Annähern der ersten Trageinrichtung (2) und der zweiten Trageinrichtung (3).

14. Abluftbehandlungssystem (1) nach einem der Ansprüche 1 bis 13,
**dadurch gekennzeichnet, dass**
beim Bilden der Kontaktelementpaare der zweite Kontaktträger (8) durch den ersten Kontaktträger (6) gegen die Federkraft (F) verschiebbar ist, oder umgekehrt.

15. Abluftbehandlungssystem (1) nach einem der Ansprüche 1 bis 14,
**dadurch gekennzeichnet, dass**
zumindest ein Material des zweiten Kontaktträgers (8) und/oder ein Material der zweiten Kontaktelemente (9) spülmaschinenfest ausgebildet ist.

16. Abluftbehandlungssystem (1) nach Anspruch 15,
**dadurch gekennzeichnet, dass**
eine Materialpaarung des Materials des zweiten Kontaktträgers (8) und des Materials der zweiten Kontaktelemente (9) spülmaschinenfest ausgebildet ist, welche Materialien vorzugsweise ein ähnliches Ausdehnungsverhalten mit der Temperatur aufweisen.

## Claims

1. An exhaust air treatment system (1), particularly for large kitchens, with at least one support member (2) and with at least one removable housing member (3) mounted or mountable on the support member, through which housing member (3) exhaust air flows or is flowable in operation of the exhaust air treatment system (1) and arranged in which housing portion (3) there is at least one UV unit (4), by which the exhaust air may be acted on by electromagnetic radiation in the UV region, wherein provided on the support member (2) and on the housing member (3) together there is at least one electrical contact device (5), **characterised in that** the electrical contact device (5) includes:
a first contact carrier (6) with a first number of first contact elements (7);
a second contact carrier (8) with a second number of second contact elements (9); and
a spring force device (10), which is constructed to act on the second contact carrier (8);
wherein at least a smaller number of the first contact elements (7) or of the second contact elements (8) are so arranged that they are moved or are movable together into electrically conductive contact with a respective contact element on the other contact carrier in pairs in order to form electrical contact pairs in a connected state of the contact device;
wherein the first contact elements (7) or the second contact elements (9) are constructed as spring contacts, the tips of which project, at least in an unconnected state of the contact device (5), beyond a surface (6a) of the contact carrier (6) in question by an excess dimension (UM), which surface (6a) is directed towards the other contact carrier (8), at least in the connected state;
wherein a spring force (F) is produced or producible by the spring force device (10) on the second contact carrier (8) in the direction towards the first contact carrier (6), when the said pairs of contact elements are formed;
wherein the first contact carrier (9) is fastened substantially immovably to a first support device (2), which first support device (2) is arranged on the support member (2) or is constituted by this support member (2);
wherein the second contact carrier (8) is fastened to a second support device (3) and is movable with respect to this second support device (3) against the spring force (F) produced or producible by the spring force device (10), which second support device (3) is arranged on the housing member (3) or is constituted by this housing member (3); and
wherein the connected state of the contact device is reached when arranging the housing member (3) on the support member (2).

2. An exhaust air treatment system (1) as claimed in Claim 1, **characterised in that** the first contact carrier (6) and the second contact carrier (8) engage one another in the connected state with at least one partial surface of their respective surfaces (6a, 8c).

3. An exhaust air treatment system (1) as claimed in Claim 2, **characterised in that** the first contact carrier (6) and/or the second contact carrier (8) has a projecting structure (8b) and/or a sealing element in the vicinity of the respective partial surface.

4. An exhaust air treatment system (1) as claimed in Claim 3, **characterised in that** the projecting structure (8b) and/or the sealing element is constructed, preferably closed, extending around the respective first (7) and/or second contact elements (9).

5. An exhaust air treatment system (1) as claimed in Claim 3 or 4, **characterised in that** the projecting structure (8b) and/or the sealing element has a height (a), in the closed state, with respect to the remaining surface (8c) of the respective contact carrier (8), which height (a) is smaller than the excess dimension (UM) of the spring contacts (7) together with a potential excess dimension of the contact elements (9) of the other contact carrier (8) with respect to its surface (8c), which surface (8c), at least in the connected state, is directed towards the surface (6a) of the contact carrier (6) carrying the spring contacts (7).

6. An exhaust air treatment system (1) as claimed in one of Claims 1 to 5, **characterised in that** the contact elements (9) which are not constructed as spring contacts have a surface substantially flush with the surface (8c) of the contact carrier (8) in question and are embedded in it.

7. An exhaust air treatment system (1) as claimed in one of Claims 1 to 6, characterised n that the first support device (2) and the second support device (3) engage one another flatly, in regions, at least in the connected state.

8. An exhaust air treatment system (1) as claimed in Claim 7, **characterised in that** the first contact carrier (6) or the second contact carrier (8) is arranged projecting with respect to a surface of the respective support device (2, 3) whilst the other contact carrier is arranged set back with respect to a surface of the respective support device.

9. An exhaust air treatment system (1) as claimed in one of Claims 1 to 8, **characterised in that** the first contact carrier (6) and the second contact carrier (8) are arranged in the vicinity of openings (2a, 3d) in the respective support device (2, 3).

10. An exhaust air treatment system (1) as claimed in Claim 8 and Claim 9, **characterised in that** folds (3e) are provided in edge regions of the respective opening (3d) on that support device (3), on which the respective contact carrier (8) is arranged projecting with respect to the surface of the support device (3) in question, which folds (3e) project in a same direction as the respective contact carrier (8).

11. An exhaust air treatment system (1) as claimed in Claim 10, **characterised in that** an excess dimension (A) of the folds (3e) with respect to the surface of the support device (3) in question is the same size as or larger than a dimension of the projection of the contact carrier (8) in question with respect to the surface of the support device (3) in question, and that an excess dimension (A) of the folds (3e) with respect to the surface of the support device (3) in question is of the same size as or smaller than a dimension of the setback of the other contact carrier (6) with respect to the surface of the other support device (2).

12. An exhaust air treatment system (1) as claimed in one of Claims 1 to 11, **characterised in that**, at least in the connected state, the first support device (2) and the second support device (3) are releasably fixed to one another.

13. An exhaust air treatment system (1) as claimed in one of Claims 1 to 12, **characterised in that** provided on the first support device (2) and/or on the second support device (3) there is at least one aligning and fixing device (17), preferably including a locking or snap device, which is constructed to hold the contact device (5) in the connected state, most preferably substantially automatically on approach of the first support device (2) and the second support device (3).

14. An exhaust air treatment system (1) as claimed in one of Claims 1 to 13, **characterised in that** when forming the pairs of contact elements the second contact carrier (8) is movable by the first contact carrier (6) against the spring force (F) or vice versa.

15. An exhaust air treatment system (1) as claimed in one of Claims 1 to 14, **characterised in that** at least one material of the second contact carrier (8) or one material of the second contact elements (9) is dishwasher safe.

16. An exhaust air treatment system (1) as claimed in Claim 15, **characterised in that** a material pairing of the material of the second carrier (8) and of the material of the second contact elements (9) is dishwasher safe, which materials preferably have a similar expansion behaviour with temperature.

## Revendications

1. Système de traitement d'air vicié (1), en particulier pour des cuisines industrielles, avec au moins une partie support (2) et avec au moins une partie carter (3) amovible, disposée ou pouvant être disposée sur la partie support (2), ladite partie carter (3) étant ou pouvant être traversée par de l'air vicié pendant le fonctionnement du système de traitement d'air vicié (1) et au moins une unité UV (4) étant disposée dans la partie carter (3), unité par laquelle l'air vicié est ou peut être sollicité par un rayonnement électromagnétique dans la plage UV, sachant qu'au moins un dispositif de contact électrique (5) est prévu conjointement sur la partie support (2) et sur la partie carter (3),
**caractérisé en ce que** le dispositif de contact électrique (5) comprend :
un premier porte-contacts (6) avec un premier nombre de premiers éléments de contact (7) ;
un deuxième porte-contacts (8) avec un deuxième nombre de deuxièmes éléments de contact (9) ;
et un dispositif (10) de force de ressort, qui est réalisé pour agir sur le deuxième porte-contacts (8) ;
sachant qu'au moins un sous-nombre des premiers éléments de contact (7) ou des deuxièmes éléments de contact (9) est disposé de telle sorte qu'ils sont ou peuvent être conjointement amenés par paires en contact électriquement conducteur avec un élément de contact respectif sur l'autre porte-contacts, afin de former dans un état relié du dispositif de contact des paires d'éléments de contact ;
sachant que les premiers éléments de contact (7) ou les deuxièmes éléments de contact (9) sont réalisés sous la forme de contacts à ressort dont les pointes, au moins dans un état non relié du dispositif de contact (5), dépassent d'une surcote (UM) d'une face supérieure (6a) du porte-contacts concerné (6), ladite face supérieure (6a) étant, au moins dans l'état relié, tournée vers l'autre porte-contacts (8) ;
sachant que le dispositif (10) de force de ressort produit ou permet de produire une force de ressort (F) sur le deuxième porte-contacts (8) en direction du premier porte-contacts (6) lorsque lesdites paires d'éléments de contact sont formées ;
sachant que le premier porte-contacts (6) est fixé pour l'essentiel sans possibilité de déplacement sur un premier moyen porteur (2), ledit premier moyen porteur (2) étant disposé sur la partie support (2) ou étant formé par cette partie support (2) ;
sachant que le deuxième porte-contacts (8) est fixé sur un deuxième moyen porteur (3) avec possibilité de déplacement par rapport à ce deuxième moyen porteur (3) à l'encontre de la force de ressort (F) produite ou pouvant être produite par le dispositif (10) de force de ressort, ledit deuxième moyen porteur (3) étant disposé sur la partie carter (3) ou étant formé par cette partie carter (3) ;
et sachant que l'état relié du dispositif de contact est atteint lorsque la partie carter (3) est disposée sur la partie support (2).

2. Système de traitement d'air vicié (1) selon la revendication 1, **caractérisé en ce que** le premier porte-contacts (6) et le deuxième porte-contacts (8), dans l'état relié, s'appliquent respectivement l'un contre l'autre par au moins une surface partielle de leurs faces supérieures respectives (6a, 8c).

3. Système de traitement d'air vicié (1) selon la revendication 2, **caractérisé en ce que** le premier porte-contacts (6) et/ou le deuxième porte-contacts (8) présentent dans la région de leur surface partielle respective une structure en saillie (8b) et/ou un élément d'étanchéité.

4. Système de traitement d'air vicié (1) selon la revendication 3, **caractérisé en ce que** la structure en saillie (8b) et/ou l'élément d'étanchéité sont formés en entourant les premiers (7) et/ou deuxièmes (9) éléments de contact respectifs, de préférence de manière fermée.

5. Système de traitement d'air vicié (1) selon la revendication 3 ou 4, **caractérisé en ce que** la structure en saillie (8b) et/ou l'élément d'étanchéité présentent dans l'état relié une hauteur (a) par rapport à une face supérieure résiduelle (8c) du porte-contacts respectif (8), ladite hauteur (a) étant inférieure à la surcote (UM) des contacts à ressort (7) augmentée d'une éventuelle surcote des éléments de contact (9) de l'autre porte-contacts (8) par rapport à sa face supérieure (8c), ladite face supérieure(8c) étant, au moins dans l'état relié, tournée vers la face supérieure (6a) du porte-contacts (6) présentant les contacts à ressort (7).

6. Système de traitement d'air vicié (1) selon l'une des revendications 1 à 5, **caractérisé en ce que** les éléments de contact (9) non réalisés sous la forme de contacts à ressort sont encastrés dans le porte-contacts concerné (8) essentiellement à fleur de la face supérieure (8c) de ce dernier.

7. Système de traitement d'air vicié (1) selon l'une des revendications 1 à 6, **caractérisé en ce que**, au moins dans l'état relié, le premier moyen porteur (2) et le deuxième moyen porteur (3) s'appliquent sectoriellement à plat l'un contre l'autre.

8. Système de traitement d'air vicié (1) selon la revendication 7, **caractérisé en ce que** le premier porte-contacts (6) ou le deuxième porte-contacts (8) est disposé en saillie par rapport à une surface du moyen porteur concerné (2, 3), tandis que l'autre porte-contacts est disposé en retrait par rapport à une surface du moyen porteur concerné.

9. Système de traitement d'air vicié (1) selon l'une des revendications 1 à 8, **caractérisé en ce que** le premier porte-contacts (6) et le deuxième porte-contacts (8) sont disposés dans la région de brèches (2a, 3d) dans le moyen porteur respectif (2, 3).

10. Système de traitement d'air vicié (1) selon la revendication 8 et la revendication 9, **caractérisé en ce que**, sur le moyen porteur (3) sur lequel le porte-contacts concerné (8) est disposé en saillie par rapport à la surface du moyen porteur concerné (3), des replis (3e) sont prévus dans les régions de bord de la brèche respective (3d), lesdits replis (3e) faisant saillie dans la même direction que le porte-contacts concerné (8).

11. Système de traitement d'air vicié (1) selon la revendication 10, **caractérisé en ce qu'**une surcote (A) des replis (3e) par rapport à la surface du moyen porteur concerné (3) est supérieure ou égale à la dimension du dépassement du porte-contacts concerné (8) par rapport à la surface du moyen porteur concerné (3),
et **en ce qu'**une surcote (A) des replis (3e) par rapport à la surface du moyen porteur concerné (3) est supérieure ou égale à la dimension du retrait de l'autre porte-contacts (6) par rapport à la surface de l'autre moyen porteur (2).

12. Système de traitement d'air vicié (1) selon l'une des revendications 1 à 11, **caractérisé en ce que**, au moins dans l'état relié, le premier moyen porteur (2) et le deuxième moyen porteur (3) sont assujettis l'un à l'autre de manière amovible.

13. Système de traitement d'air vicié (1) selon l'une des revendications 1 à 12, **caractérisé en ce que**, sur le premier moyen porteur (2) et/ou sur le deuxième moyen porteur (3), il est prévu au moins un dispositif (17) d'alignement et d'assujettissement, de préférence comprenant un dispositif de crantage ou d'enclenchement, qui est réalisé pour maintenir le dispositif de contact (5) dans l'état relié, d'une manière la plus préférée pour l'essentiel automatiquement lors du rapprochement du premier moyen porteur (2) et du deuxième moyen porteur (3).

14. Système de traitement d'air vicié (1) selon l'une des revendications 1 à 13, **caractérisé en ce que**, lors de la formation des paires d'éléments de contact, le deuxième porte-contacts (8) peut être déplacé par le premier porte-contacts (6) à l'encontre de la force de ressort (F), ou inversement.

15. Système de traitement d'air vicié (1) selon l'une des revendications 1 à 14, **caractérisé en ce qu'**au moins un matériau du deuxième porte-contacts (8) et/ou un matériau des deuxièmes éléments de contact (9) est réalisé comme étant résistant au lave-vaisselle.

16. Système de traitement d'air vicié (1) selon la revendication 15, **caractérisé en ce qu'**un appariement du matériau du deuxième porte-contacts (8) et du matériau des deuxièmes éléments de contact (9) est réalisé comme étant résistant au lave-vaisselle, lesdits matériaux présentant de préférence un comportement similaire de dilatation en fonction de la température.
